# EUROPEAN PATENT SPECIFICATION

(11) **EP 4 159 745 B1**
(45) Date of publication and mention of the grant of the patent: **24.09.2025**
(21) Application number: 21816810.2
(22) Date of filing: 26.05.2021
(51) Int. Cl.: C07K 7/06, A61K 38/08, A61P 1/04, A61P 3/00, A61P 17/00, A61P 25/18, A61P 29/00, A61P 31/14, A61P 35/04, A61P 37/08, A61P 43/00, C07K 19/00

(54) **S100A8-INHIBITING PEPTIDE AND THERAPEUTIC DRUG FOR DISEASE WHICH CONTAINING SAME**
S100A8-HEMMENDES PEPTID UND THERAPEUTISCHES ARZNEIMITTEL FÜR KRANKHEITEN DAMIT
PEPTIDE INHIBITEUR DE S100A8 ET MÉDICAMENT THÉRAPEUTIQUE CONTRE UNE MALADIE LE CONTENANT

(30) Priority: 02.06.2020 JP 2020096367
(43) Date of publication of application: 05.04.2023
(73) Proprietor: Tokyo Women's Medical University, Tokyo 162-8666 (JP)
(72) Inventor: MARU, Yoshiro, Tokyo 162-8666 (JP); DEGUCHI, Atsuko, Tokyo 162-8666 (JP); NISHIKAWA, Kiyotaka, Kyotanabe-shi, Kyoto 610-0394 (JP); TAKAHASHI, Miho, Kyotanabe-shi, Kyoto 610-0394 (JP); OHTO, Umeharu, Tokyo 113-8654 (JP)
(74) Representative: Dehns
(86) International application number: PCT/JP2021/020095
(87) International publication number: WO 2021/246265

(56) References cited:
- WO-A2-2009/038756
- JP-A- 2020 083 759
- DEGUCHI ATSUKO ET AL: "Novel multivalent S100A8 inhibitory peptides attenuate tumor progression and metastasis by inhibiting the TLR4-dependent pathway", vol. 30, no. 7, 17 March 2023 (2023-03-17), New York, pages 973 - 984, XP093149781, ISSN: 0929-1903, Retrieved from the Internet <URL:https://www.nature.com/articles/s41417-023-00604-3> DOI: 10.1038/s41417-023-00604-3
- DEGUCHI A; TOMITA T; OHTO U; TAKEMURA K; KITAO A; AKASHI-TAKAMURA S; MIYAKE K; MARU Y: "Eritoran inhibits S100A8-mediated TLR4/MD-2 activation and tumor growth by changing the immune microenvironment", ONCOGENE, NATURE PUBLISHING GROUP UK, LONDON, vol. 35, no. 11, 1 January 1900 (1900-01-01), London , pages 1445 - 1456, XP037324348, ISSN: 0950-9232, DOI: 10.1038/onc.2015.211
- BERTHIER SYLVIE, NGUYEN MINH VU CHUONG, BAILLET ATHAN, HOGRAINDLEUR MARC-ANDRÉ, PACLET MARIE-HÉLÈNE, POLACK BENOÎT, MOREL FRANÇOIS: "Molecular Interface of S100A8 with Cytochrome b558 and NADPH Oxidase Activation", PLOS ONE, vol. 7, no. 7, 10 July 2012 (2012-07-10), pages e40277, XP055880554, DOI: 10.1371/journal.pone.0040277

## Description

### Technical Field

The present invention relates to S100A8-inhibiting peptides and a disease therapeutic agent containing the same.

### Background Art

Currently, a top cause of death in our country is cancer-related death, and many of them are metastasis to distant organs. In the process of cancer development, it has been found that there is an inflammation-like microenvironment present at the surrounding tumors together with abnormal activation associated with mutation at gene level in cancer cells themselves.

As described above, it is suggested that the microenvironment caused by inflammation plays an important role, and the present inventor has confirmed the presence of pre-metastatic lung microenvironment (Non-Patent Literature 1). In addition, the present inventor has identified S100A8 (S100 calcium binding protein A8) as a pre-metastatic lung microenvironment forming factor in lung metastasis, and has reported that S100A8 enhances vascular permeability and promotes lung metastasis of cancer (Non-Patent Literature 2). Furthermore, the present inventor has reported that S100A8 acts as a toll-like receptor 4 (TLR4) endogenous ligand, that the TLR4 inhibitor Eritoran remarkably suppresses the development of a subcutaneous tumor and inhibits both the recruitment of myeloid cells and tumor angiogenesis, that the Carboxyl-terminal side of S100A8 is involved in binding between a TLR4/MD-2 complex and S100A8, and the like (Non-Patent Literature 3).

### Citation List

### Patent Literature

Patent Literature 1: JP 5718574 B2
Patent Literature 2: JP 2020 083759 A describes a functional peptide usable, for example, for the treatment of colitis.
Patent Literature 3: WO 2009/038756 A2 describes LINE polypeptides; and compositions, including immunogenic compositions, comprising a subject LINE polypeptide.

### Non-Patent Literature

Non-Patent Literature 1: Hiratsuka et al., Nat Cell Biol. 8, 1369-1375, 2006
Non-Patent Literature 2: Hiratsuka et al., Nat Commun,4.1853, 2013
Non-Patent Literature 3: Deguchi et al., Oncogene 35, 1445-1456, 2016.
Non-Patent Literature 4: Berthier et al., PLoS One, 7(7):e40277, 2012 describes the molecular interface of S100A8 with cytochrome b558 and NADPH oxidase activation.

### Summary of Invention

### Technical Problem

From the findings found so far, it is expected that if S100A8 can be inhibited, it acts on both cancer microenvironment and pre-metastatic microenvironment and can suppress development of cancer, but no peptide that directly inhibits S100A8 has been found so far.

The present invention has been made in view of the above circumstances, and an object thereof is to provide novel peptides capable of inhibiting S100A8 and a disease therapeutic agent containing the peptide.

### Solution to Problem

In order to solve the above problem, the S100A8-inhibiting peptide of the present invention consists of:
(A) a peptide consisting of 5 to 10 residues in length contained within an amino acid sequence of SEQ ID NO: 1 comprising an alanine (Ala) located at the fifth position from the N-terminus in an amino acid sequence of amino acid sequence of SEQ ID NO: 1, or
(B) a peptide consisting of an amino acid sequence of SEQ ID NO: 2.

The disease therapeutic agent of the present invention is characterized by containing the S100A8-inhibiting peptide.

### Advantageous Effects of Invention

Since the S100A8-inhibiting peptide of the present invention binds to a TLR4/MD-2 complex with high affinity, it can inhibit the activity of S100A8. Therefore, the composition containing the S100A8-inhibiting peptide of the present invention acts on cancer microenvironment and pre-metastatic microenvironment, and can suppress development of cancer, and thus is useful as a therapeutic agent against metastatic cancer or the like. In addition, the disease therapeutic agent containing the S100A8-inhibiting peptide of the present invention can treat other diseases (psychiatric diseases, lifestyle-related diseases, rheumatoid arthritis, 2019 novel coronavirus infection (COVID-19), Crohn's disease, ulcerative colitis, cystic fibrosis, allergic dermatitis) in which S100A8 is involved, and the like.

### Brief Description of Drawings

Fig. 1 is a diagram showing amino acid sequences of Peptides#1 to #6 and binding affinity of each peptide to TLR4/MD-2.
Fig. 2 is a diagram showing results of verifying competitive inhibitory activity of Peptides#1 to #6 on binding of S100A8 to a TLR4/MD-2 complex by ELISA method (A) and immunoprecipitation method (B).
Fig. 3 is a diagram showing affinity for TLR4/MD-2 of Peptide3A1 to Peptide3A10 in which each amino acid of Peptide3 is substituted with alanine.
Fig. 4 is a diagram showing influences of divalent Peptide3 and bivalent Peptide3A5 on expression of mRNA of IL-8.
Fig. 5 is a diagram showing competitive inhibitory activity of monovalent Peptide3A5 or divalent Peptide3A5 on binding of S100A8 to a TLR4/MD-2 complex.
Fig. 6 is a view showing an effect on a mouse subcutaneous tumor by administration of divalent Peptide3A5.
Fig. 7(A) is a diagram showing affinity of each peptide to TLR4/MD-2. Fig. 7(B) is a diagram showing competitive inhibitory activity of each peptide on binding activity of S100A8 to a TLR4/MD-2 complex.
Fig. 8 shows results of competition assays for divalent ILVIK, divalent Peptide3A5, and tetravalent ILVIK.
Fig. 9 is diagrams showing influences of divalent ILVIK on mouse subcutaneous tumors.
Fig. 10 is a diagram showing influences of tetravalent ILVIK on mouse subcutaneous tumors.

### Description of Embodiments

An embodiment the S100A8-inhibiting peptide (S100 calcium binding protein A8 - inhibiting peptide) of the present invention will be described.

The S100A8-inhibiting peptide of the present invention includes:
(A) a peptide of 5 to 10 residues in length containing the fifth alanine (Ala) from the N-terminus in an amino acid sequence of SEQ ID NO: 1, or
(B) a peptide consisting of an amino acid sequence of SEQ ID NO: 2.
   SEQ ID NO: 1: Phe-Gln-Glu-Phe-Ala-Ile-Leu-Val-Ile-Lys (FQEFAILVIK)
   SEQ ID NO: 2: Ile-Leu-Val-Ile-Lys (ILVIK)
      Specifically, the peptide (A) is preferably a 10-residue peptide consisting of an amino acid sequence of SEQ ID NO: 1. Examples of other peptides include peptides of the following SEQ ID NOs: 3 to 12 and the like.
   SEQ ID NO: 3: Phe-Gln-Glu-Phe-Ala-Ile-Leu-Val-Ile (FQEFAILVI)
   SEQ ID NO: 4: Gln-Glu-Phe-Ala-Ile-Leu-Val (QEFAILV)
   SEQ ID NO: 5: Glu-Phe-Ala-Ile-Leu-Val-Ile (EFAILVI)
   SEQ ID NO: 6: Glu-Phe-Ala-Ile-Leu-Val (EFAILV)
   SEQ ID NO: 7: Phe-Ala-Ile-Leu-Val-Ile (FAILVI)
   SEQ ID NO: 8: Phe-Ala-Ile-Leu-Val (FAILV)
   SEQ ID NO: 9: Ala-Ile-Leu-Val-Ile (AILVI)
   SEQ ID NO: 10: Ala-Ile-Leu-Val-Ile-Lys (AILVIK)
   SEQ ID NO: 11: Phe-Gln-Glu-Phe-Ala-Ile-Leu-Val (FQEFAILV)
   SEQ ID NO: 12: Gln-Glu-Phe-Ala-Ile-Leu-Val-Ile (QEFAILVI)

In addition, the S100A8-inhibiting peptide of the present invention is preferably a divalent peptide containing two identical peptides among the peptides (A) and (B). Furthermore, the S100A8-inhibiting peptide of the present invention is preferably a tetravalent peptide containing four identical peptides among the peptides (A) and (B).

Forms and synthesis methods of the bivalent peptide or the tetravalent peptide are not particularly limited. Specifically, for example, regarding synthesis of a peptide, condensation and removal of a protecting group can be performed according to a conventionally known method. For example, a peptide solid phase synthesis method, an Fmoc peptide synthesis method or the like can be adopted, and the synthesis can also be performed by a commercially available peptide synthesizer.

Preferably, the peptide synthesis method on a sheet already proposed by the present inventor (Patent Literature 1) can be considered. A synthetic core structure of a divalent peptide or a synthetic core structure of a tetravalent peptide can be formed from an amino group on a sheet of cellulose, resin or the like. Specifically, for example, Fmoc-Lys and Fmoc-His represented by Chemical Formula 1 shown below can be used to form a divalent or tetravalent synthetic core structure. In consideration of interactions with other amino acids and the like, Fmoc-Lys is preferable. A branch point can be provided by introducing Fmoc-Lys, and amino acid synthesis from the branch point can be designed to be divalent.

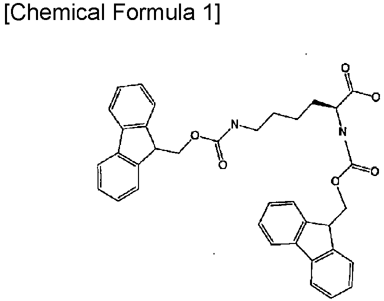

In addition, in the case of forming a tetravalent synthetic core structure, it is considered that peptide Fmoc-Lys is continuously synthesized twice. The synthetic core structure is not specifically limited as long as it is a structure capable of extending a divalent or tetravalent peptide.

Furthermore, a spacer can be appropriately introduced into the divalent peptide or the tetravalent peptide. The spacer is not specifically limited, and for example, Ahx (amino hexanoic acid [NH₂-(CH₂)₅-COOH]) can be exemplified. For cutting out the synthetic peptide, for example, trifluoroacetic acid or the like can be appropriately used.

In addition, when the S100A8-inhibiting peptide of the present invention is in the form of a tetravalent peptide, the following molecular core structure (Chemical Formula 2) formed by binding three lysines (Lys) can be included.

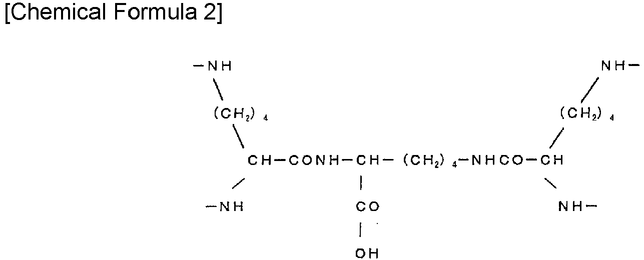

The S100A8-inhibiting peptide of the present invention can be exemplified by a tetravalent peptide in which the peptide (A) or (B) is bonded to each of the four amino groups located at the end of the above molecular core structure directly or via a spacer.

As a more preferred embodiment, for example, in Chemical Formula 3 shown below, a tetravalent peptide in which any one of the peptides (A) or (B) is incorporated into each of four XXXX parts located at the end of the molecular core structure composed of three lysines (Lys) is exemplified.

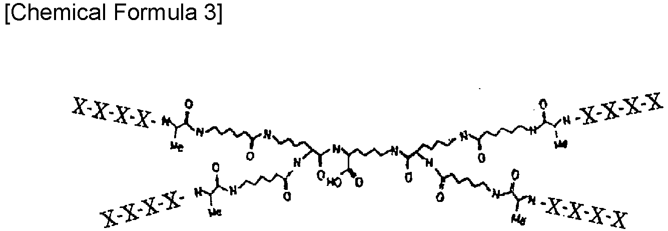

In Chemical Formula 3, the position at which the peptide (A) or (B) is incorporated is described as "XXXX" for convenience.

In addition, Chemical Formula 3 exemplifies a form in which a spacer is bonded to each of the four amino groups located at the end of the molecular core structure, but the peptide (A) or (B) can also be directly bonded to each of the four amino groups without interposing a spacer. When the spacer is bound, specific molecules and lengths are not limited as long as a binding property to the TLR4/MD-2 complex is not impaired, and they can be appropriately designed. As the spacer, for example, a spacer having an amino acid at the terminal and a chain length of about 4 to 10 carbon atoms is preferable, and in particular, amino hexanoic acid [NH₂-(CH₂)₅-COOH] (aminocaproic acid) represented in Chemical Formula 3 can be preferably exemplified. Also, examples of the amino acid contained in the spacer include alanine (A).

This form of the S100A8-inhibiting peptide (tetravalent peptide) can also be prepared by a known method such as using a peptide synthesizing device or the like. For example, the incorporated peptide (A) or (B) can be synthesized by sequentially adding amino acids to the tetravalent core structure, and can be conveniently bulk synthesized by the same method as that for monovalent peptide synthesis.

In addition, the S100A8-inhibiting peptide may have a modified molecule at each terminal of the peptide incorporated into the XXXX part of Chemical Formula 3. Since a positive charge is generated when NH₂ is exposed at the terminal of the peptide, from the viewpoint of charge regulation, it is also considered that a molecule having no charge or a hydrophobic molecule is bound to each terminal as a modified molecule. In addition, when a therapeutic agent containing the S100A8-inhibiting peptide of the present invention is orally administered, NH₂ at the terminal can also be protected by an acetyl group for the purpose of stabilization for suppressing degradation by protease in the gastrointestinal tract. As described above, the modified molecule at the terminal of the peptide (A) or (B) can be appropriately selected according to a desired effect, and may be modified by phosphorylation, methylation, adenylation, sugar chain addition, or the like.

Furthermore, the S100A8-inhibiting peptide can also include, for example, a basic membrane penetrating sequence including amino acids such as histidine (His), lysine (Lys), arginine (Arg), and the like.

The S100A8-inhibiting peptide of the present invention binds to the TLR4/MD-2 complex with high affinity. That is, the S100A8-inhibiting peptide of the present invention has excellent competitive activity for binding between the TLR4/MD-2 complex and S100A8. Therefore, the composition containing the S100A8-inhibiting peptide of the present invention acts on cancer microenvironment and pre-metastatic microenvironment, and can suppress development of cancer, and thus is useful as a therapeutic agent for metastatic cancer.

In addition, it has been reported so far that S100A8 is involved not only in cancer diseases but also in overexpression of S100A8 in synovial fluid of patients with rheumatoid arthritis (Odink et al., Nature 330, 80, 1987), macrophage-derived S100A8 controls activation of adipocytes (Sekimoto, et al., PNAS, 112, E2058, 2015), activation of innate immunity via TLR4 induces depression-like behavior (Nie, et al., Neuron, 99, 464, 2018), increased expression of S100A8 is observed in bronchoalveolar lavage fluid of patients infected with COVID-19 (Zhou et al., Cell Host and Microbe, 2020), and the like. Therefore, the disease therapeutic agent containing the S100A8-inhibiting peptide of the present invention can treat diseases such as metastatic cancer, psychiatric diseases, lifestyle diseases, rheumatoid arthritis, COVID-19, Crohn's disease, ulcerative colitis, cystic fibrosis, and allergic dermatitis.

An administration form of the disease therapeutic agent containing the S100A8-inhibiting peptide of the present invention is not particularly limited, and may be oral administration or parenteral administration. Examples of the parenteral administration include injection administration such as intramuscular injection, intravenous injection, subcutaneous injection, percutaneous administration, and transmucosal administration (nasal, oral, ocular, pulmonary, vaginal, rectal), and the like.

The disease therapeutic agent of the present invention may use the S100A8-inhibiting peptide as it is as an active ingredient, or may be formulated by adding a pharmaceutically acceptable carrier, excipient, additive, or the like. Examples of the dosage form include liquid preparations (for example, injections), dispersions, suspensions, tablets, pills, powders, suppositories, powders, granules, granules, capsules, syrups, troches, inhalants, ointments, eye drops, nasal drops, ear drops, cataplasms, and the like.

The disease therapeutic agent of the present invention can be formulated by a conventional method using, for example, an excipient, a binder, a disintegrant, a lubricant, a dissolving agent, a solubilizing agent, a coloring agent, a flavoring agent, a stabilizing agent, an emulsifier, an absorption promoting agent, a surfactant, a pH adjusting agent, an antiseptic agent, an antioxidant and the like as appropriate.

Examples of the component to be used for formulation include purified water, saline, phosphate buffer, dextrose, pharmaceutically acceptable organic solvents such as glycerol and ethanol, animal and vegetable oils, lactose, mannitol, glucose, sorbitol, crystalline cellulose, hydroxypropyl cellulose, starch, corn starch, silicic anhydride, magnesium aluminum silicate, collagen, polyvinyl alcohol, polyvinyl pyrrolidone, carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohol, stearyl alcohol, stearic acid, human serum albumin, and the like.

The disease therapeutic agent of the present invention can be administered to mammals including humans (for example, mouse, rat, guinea pig, rabbit, dog, horse, monkey, pig, and the like), and in particular, the dosage when administered to humans varies depending on the symptoms, age, sex, weight and sensitivity difference of the patient, administration method, administration interval, type of active ingredient, and type of formulation, and is not particularly limited, but for example, 100 µg to 1000 mg can be administered once or in several divided doses.

The S100A8-inhibiting peptide and the disease therapeutic agent involving S100A8 of the present invention are not limited to the above embodiments, and can be appropriately designed as long as the binding property to the TLR4/MD-2 complex and the S100A8 inhibitory effect are not impaired.

### Examples

Hereinafter, the present invention will be described with reference to Examples, but the S100A8-inhibiting peptide and the disease therapeutic agent of the present invention are not limited to the following Examples at all.

### <Example 1> Identification of binding region between human S100A8 and TLR4/MD-2 complex

### (1) Synthesis of overlapping peptide

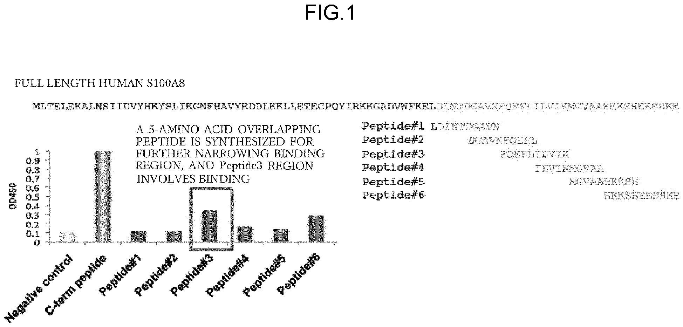

So far, it has been reported that mouse S100A8 binds to a TLR4/MD-2 complex on the carboxyl terminal side (Non-Patent Literature 3), and based on this report, full length human S100A8 was divided into three parts, and it was revealed that the carboxyl terminal side binds to the TLR4/MD-2 complex as with mouse S100A8 (Fig. 1). Furthermore, for the purpose of narrowing the binding region with the TLR4/MD-2 complex, a peptide of 10 amino acids in the carboxyl terminal amino acid sequence was synthesized. At this time, the sequence can be comprehensively analyzed by overlapping by five amino acids. The synthesized peptides were immobilized on an ELISA plate (Thermo Fisher Scientific Inc.; Maxisorp), and then non-specific binding was blocked with a blocking buffer. Each well was washed with PBS-Tween 20 and then incubated with a high-purity human TLR4/MD-2 recombinant protein, then each well was incubated with an anti-TLR4 antibody (Abcam) and an anti-rabbit IgG-HRP labeled antibody (GE Healthcare), and finally a reaction with TMB as an HRP substrate was terminated with an equivalent of 1 N sulfuric acid. The amount of bound TLR4/MD-2 was quantified by measuring the absorbance at OD 450 nm.

As a result, Peptide#3 (SEQ ID NO: 13: Phe-Gln-Glu-Phe-Leu-Ile-Leu-Val-Ile-Lys (FQEFLILVIK)) was found to have high affinity for TLR4/MD-2 (Fig. 1).

### (2) Examination of competitive inhibitory activity of each peptide on binding of S100A8 to TLR4/MD-2 complex using ELISA

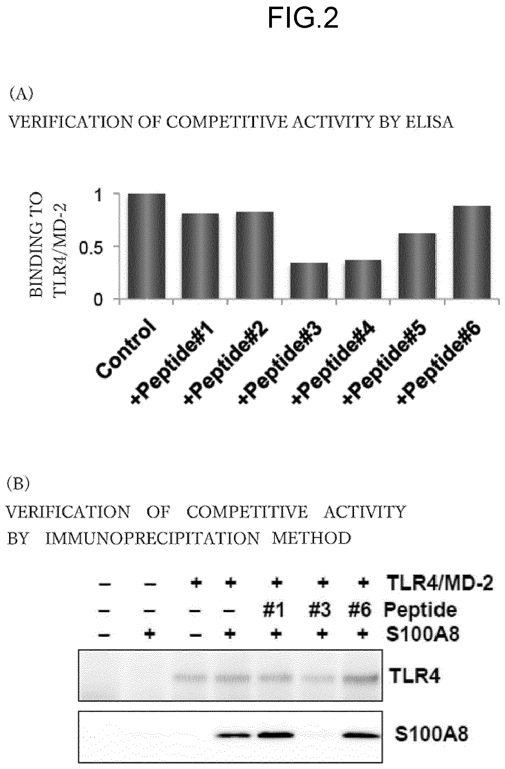

Pre-purified S100A8 protein was immobilized on an ELISA plate. Non-specific binding was blocked with blocking buffer in the same manner as in Example 1. Peptides #1 to #6 were added simultaneously with TLR4/MD-2, and the mixture was reacted. Each well was washed with PBS-Tween 20, then incubated with an anti-TLR4 antibody (Abcam) and an anti-rabbit IgG-HRP labeled antibody (GE Healthcare) subsequently, and finally a reaction with TMB as an HRP substrate was terminated with an equivalent of 1 N sulfuric acid. Bound TLR4/MD-2 was quantified by measuring the absorbance at OD 450 nm. Peptide#3 and Peptide#4 were found to have competitive inhibitory activity on the binding between S100A8 and the TLR4/MD-2 complex (Fig. 2A).

### (3) Examination of competitive inhibitory activity of each peptide on binding of S100A8 to TLR4/MD-2 complex using immunoprecipitation method

The TLR4/MD-2 complex and S100A8 were mixed in a tube. At this time, 10 times the molar amount of each of Peptides#1 to #6 was added, and immunoprecipitation was performed with an anti-TLR4 antibody. The immunoprecipitate was washed several times with a washing buffer, and the final pellet was electrophoresed by SDS-PAGE. Then, S100A8 and TLR4 were detected by Western blotting using an anti-S100A8 antibody and an anti-TLR4 antibody. As a result, it became clear that S100A8 was not detected only in the presence of Peptide#3, that is, Peptide#3 had competitive inhibitory activity on the binding of S100A8 to the TLR4/MD-2 complex (Fig. 2B).

### <Example 2> Identification of high affinity peptide by alanine scanning based on binding to TLR4/MD-2 complex as index

Peptide3A1, Peptide3A2, Peptide3A3, Peptide3A4, Peptide3A5, Peptide3A6, Peptide3A7, Peptide3A8, Peptide3A9, and Peptide3A10 in which each amino acid of Peptide#3 (SEQ ID NO: 13: Phe-Gln-Glu-Phe-Leu-Ile-Leu-Val-Ile-Lys) was substituted with alanine were synthesized. In these peptides, the description of "A1 to A10" indicates a position from the N-terminal side in an amino acid sequence of SEQ ID NO: 13, that is, a position substituted with alanine.

In addition, acetylation was introduced at the amino terminus of these peptides, and amidation modification was introduced at the carboxyl terminus. These 10 peptides were immobilized on an ELISA plate (Thermo Fisher Scientific Inc.; Maxisorp) to verify binding with TLR4/MD-2.

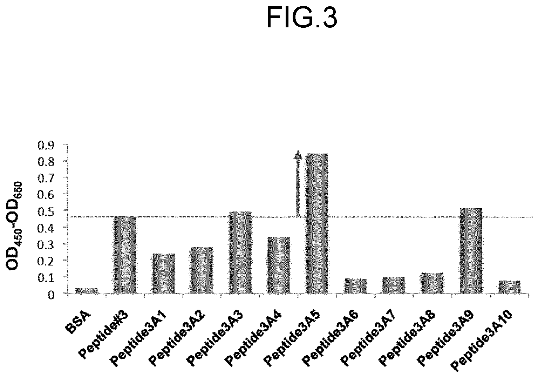

The results are shown in Fig. 3.

Peptide3A5 (SEQ ID NO: 1: Phe-Gln-Glu-Phe-Ala-Ile-Leu-Val-Ile-Lys) had higher affinity for TLR4/MD-2 than Peptide#3, the original sequence derived from S100A8. On the other hand, binding of Peptide3A6, Peptide3A7, Peptide3A8, and Peptide3A10 to TLR4/MD-2 was reduced.

### <Example 3> Inhibition of IL-8 induction by the divalent peptide (Peptide3A5)

Human colorectal cancer SW480 cells express TLR4, and induce IL-8 by S100A8 stimulation. In order to verify inhibitory activity of peptides isolated so far, after pretreating divalent Peptide#3 or divalent Peptide3A5, the cells were stimulated with S100A8 recombinant protein, and the mRNA expression of IL-8 after 5 hours was analyzed. The divalent Peptide#3 and the divalent Peptide3A5 were prepared according to the method of Patent Literature 1 described above.

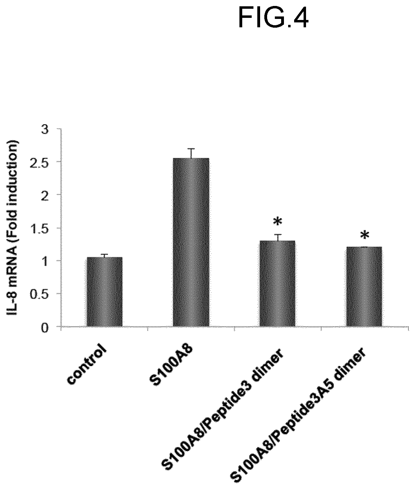

As a result, it was confirmed that the divalent peptides of Peptide#3 and Peptide3A5 remarkably inhibited the expression of mRNA of IL-8 induced by S100A8 (Fig. 4).

### <Example 4> Competitive inhibitory activity of divalent Peptide3A5 on binding of S100A8 to TLR4/MD-2 complex

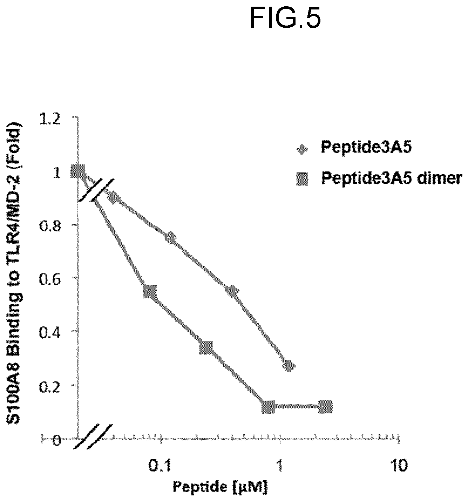

In order to verify whether the competitive inhibitory activity changes in the case of a multivalent type, first, S100A8 was immobilized on an ELISA plate, then monovalent Peptide3A5 and divalent Peptide3A5 were added at various concentrations simultaneously with the TLR4/MD-2 complex, and the amount of TLR4/MD-2 bound to S100A8 was measured to verify competitive inhibitory activity of each peptide. As a result, it was found that the divalent Peptide3A5 showed the competitive inhibitory activity at a lower concentration as compared with the monovalent type (Fig. 5).

### <Example 5> Evaluation of antitumor activity of divalent Peptide3A5 in Xenograft model

As shown in Fig. 5, since the competitive inhibitory activity of the divalent peptide is higher than that of the monovalent peptide, the divalent Peptide3A5 was used in an animal experiment.

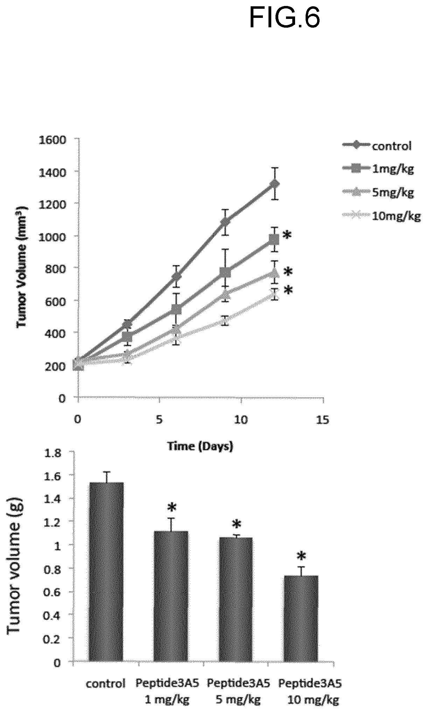

First, two weeks after transplantation of SW480 cells subcutaneously in a mouse, the size of the subcutaneous tumor was measured, and confirmed to be equivalent tumor size. The peptide was administered to the abdominal cavity at the indicated concentrations twice a week, and the size of the tumor was measured over time. After two weeks, the size of the subcutaneous tumor was measured, and the subcutaneous tumor was collected. As shown in Fig. 6, the divalent Peptide3A5 suppressed the development of subcutaneous tumor in mice in a concentration-dependent manner.

### <Example 6> Identification of S100A8-inhibiting peptide by chain length screening based on divalent Peptide3A5

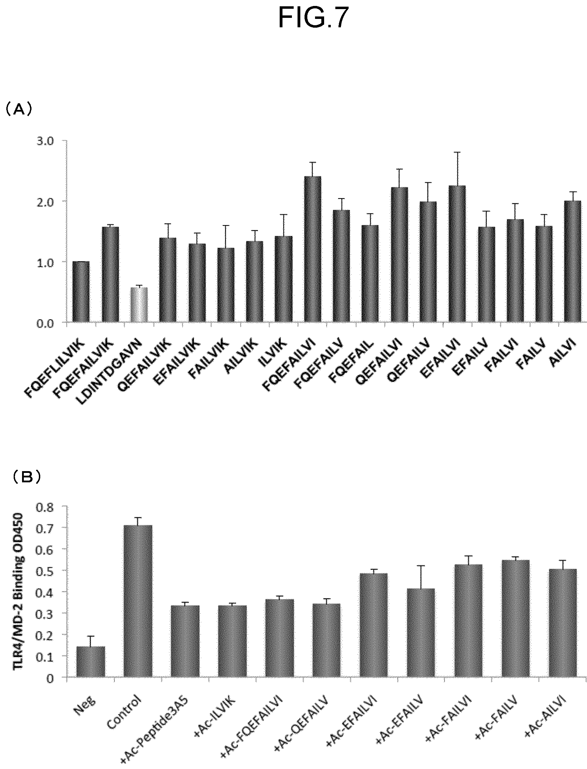

In order to identify an essential region in the Peptide3A5 sequence, a series of partial sequence peptides based on the amino acid sequence of Peptide3A5, which were obtained by deleting the amino acid from the amino terminal side or the carboxyl terminal side, were spot-synthesized as divalent peptides on a cellulose membrane sheet using the method of Patent Literature 1. Several kinds of affinity peptides with high affinity to TLR4/MD-2 were obtained by reacting the sheet with TLR4/MD-2 (Fig. 7A). Candidate peptides were subjected to a competition assay similar to Example 1 (Fig. 2).

### The results are shown in Fig. 7.

In Fig. 7(B), "neg" indicates binding between bovine serum albumin and TLR4/MD-2, and "Control" indicates binding between S100A8 and TLR4/MD-2.

As shown in Fig. 7, it was confirmed that all peptides of the following SEQ ID NOs: 1 to 12 had excellent affinity for TLR4/MD-2.
SEQ ID NO: 1: Phe-Gln-Glu-Phe-Ala-Ile-Leu-Val-Ile-Lys (FQEFAILVIK)
SEQ ID NO: 2: Ile-Leu-Val-Ile-Lys (ILVIK)
SEQ ID NO: 3: Phe-Gln-Glu-Phe-Ala-Ile-Leu-Val-Ile (FQEFAILVI)
SEQ ID NO: 4: Gln-Glu-Phe-Ala-Ile-Leu-Val (QEFAILV)
SEQ ID NO: 5: Glu-Phe-Ala-Ile-Leu-Val-Ile (EFAILVI)
SEQ ID NO: 6: Glu-Phe-Ala-Ile-Leu-Val (EFAILV)
SEQ ID NO: 7: Phe-Ala-Ile-Leu-Val-Ile (FAILVI)
SEQ ID NO: 8: Phe-Ala-Ile-Leu-Val (FAILV)
SEQ ID NO: 9: Ala-Ile-Leu-Val-Ile (AILVI)
SEQ ID NO: 10: Ala-Ile-Leu-Val-Ile-Lys (AILVIK)
SEQ ID NO: 11: Phe-Gln-Glu-Phe-Ala-Ile-Leu-Val (FQEFAILV)
SEQ ID NO: 12: Gln-Glu-Phe-Ala-Ile-Leu-Val-Ile (QEFAILVI)
   Among these peptides, as a candidate having both a high affinity and a competitive inhibitory activity,
   SEQ ID NO: 2: Ile-Leu-Val-Ile-Lys (ILVIK)
   SEQ ID NO: 3: Phe-Gln-Glu-Phe-Ala-Ile-Leu-Val-Ile (FQEFAILVI)
   SEQ ID NO: 4: Gln-Glu-Phe-Ala-Ile-Leu-Val (QEFAILV)
were identified. ILVIK (SEQ ID NO: 2) was identified as a candidate because in the verification using a monovalent form binding was reduced when the carboxyl-terminal lysine (K) was changed to alanine, and there was a high possibility that synthesis of long chain was difficult.

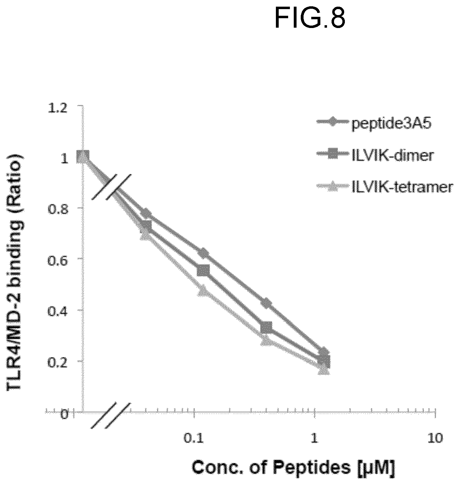

A competition assay was performed using divalent ILVIK (SEQ ID NO. 2), divalent Peptide3A5 (SEQ ID NO. 1), and tetravalent ILVIK. The tetravalent ILVIK was also synthesized using the method of Patent Literature 1. As shown in Fig. 8, it became clear that the divalent ILVIK exhibits almost similar competitive activity as the divalent Peptide3A5. The tetravalent ILVIK showed almost the same competitive activity as the divalent ILVIK.

### <Example 7> Evaluation of antitumor activity in Xenograft model of divalent ILVIK

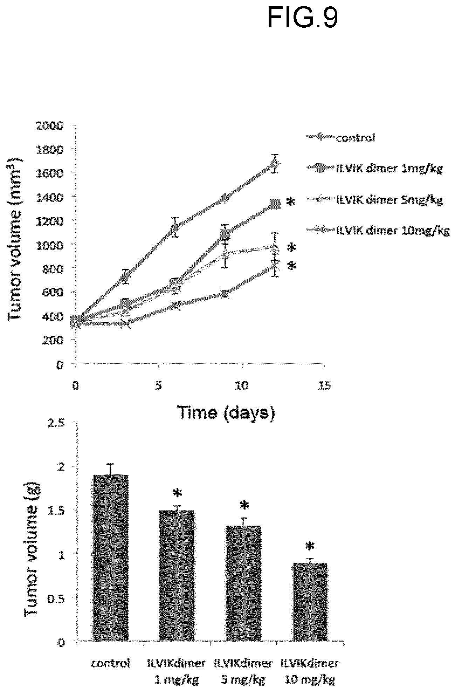

The divalent ILVIK was identified as an S100A8-inhibiting peptide candidate by chain length screening, and influence of the divalent ILVIK on mouse subcutaneous tumors was examined in the same manner as in Example 5 (Fig. 6). First, two weeks after transplantation of SW480 cells subcutaneously in a mouse, the size of the subcutaneous tumor is measured, and confirmed to be equivalent tumor size. The peptide was administered to the abdominal cavity at the indicated concentrations twice a week, and the size of the tumor was measured over time. After two weeks, the size of the subcutaneous tumor was measured, and the subcutaneous tumor was collected. The divalent ILVIK suppressed the development of mouse subcutaneous tumor in a concentration-dependent manner (Fig. 9).

### <Example 8> Evaluation of antitumor activity in Xenograft model of tetravalent ILVIK

Since it was found that proliferation of subcutaneous tumors was suppressed by the divalent ILVIK which was obtained as an S100A8-inhibiting peptide candidate by chain length screening, an effect of tetravalent ILVIK was further examined in the same manner as in Example 5 (Fig. 6). The tetravalent ILVIK was synthesized using the method of Patent Literature 1.

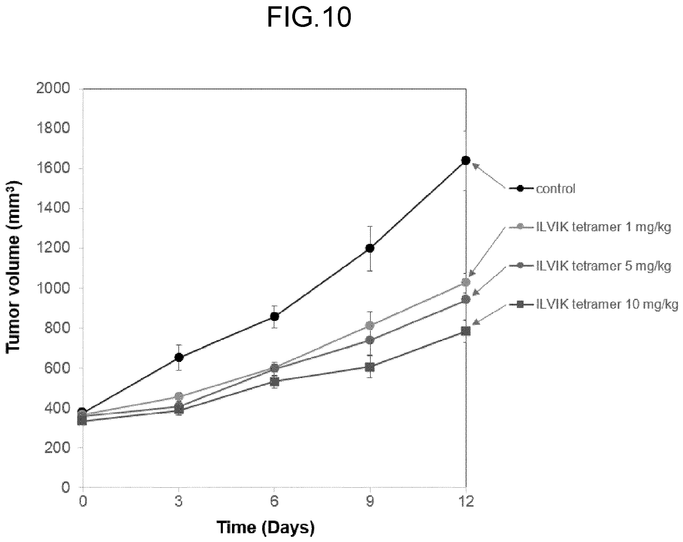

First, two weeks after transplantation of SW480 cells subcutaneously in a mouse, the size of the subcutaneous tumor was measured, and confirmed to be equivalent tumor size. The tetravalent ILVIK was administered to the abdominal cavity at the indicated concentrations (1 mg/kg, 5 mg/kg, 10 mg/kg) twice a week, and the size of the tumor was measured over time. After two weeks, the size of the subcutaneous tumor was measured, and the subcutaneous tumor was collected. The tetravalent ILVIK suppressed the development of mouse subcutaneous tumors in a concentration-dependent manner (Fig. 10).

## Claims

1. A S100A8-inhibiting peptide consisting of:
(A) a peptide consisting of 5 to 10 residues in length contained within an amino acid sequence of SEQ ID NO:1, comprising an alanine (Ala) located at the fifth position from the N-terminus in an amino acid sequence of SEQ ID NO: 1, or
(B) a peptide consisting of an amino acid sequence of SEQ ID NO: 2.

2. The S100A8-inhibiting peptide according to claim 1, wherein the peptide (A) consists of an amino acid sequence selected from SEQ ID NOs: 1 and 3 to 12.

3. The S100A8-inhibiting peptide according to claim 1, wherein the peptide (A) consists of the amino acid sequence of SEQ ID NO: 1.

4. A bivalent S100A8-inhibiting peptide containing two identical peptides among the peptides (A) and (B):
(A) a peptide consisting of 5 to 10 residues in length contained within an amino acid sequence of SEQ ID NO:1, comprising an alanine (Ala) located at the fifth position from the N-terminus in an amino acid sequence of SEQ ID NO: 1,
(B) a peptide consisting of an amino acid sequence of SEQ ID NO: 2.

5. A tetravalent S100A8-inhibiting peptide containing four identical peptides among the peptides (A) and (B):
(A) a peptide consisting of 5 to 10 residues in length contained within an amino acid sequence of SEQ ID NO:1, comprising an alanine (Ala) located at the fifth position from the N-terminus in an amino acid sequence of SEQ ID NO: 1,
(B) a peptide consisting of an amino acid sequence of SEQ ID NO: 2.

6. A disease therapeutic agent, comprising the S100A8-inhibiting peptide according to any one of claims 1 to 5.

7. The disease therapeutic agent according to claim 6, wherein the disease is at least one of metastatic cancer, psychiatric diseases, lifestyle diseases, rheumatoid arthritis, COVID-19, Crohn's disease, ulcerative colitis, cystic fibrosis, and allergic dermatitis.

## Patentansprüche

1. S100A8-hemmendes Peptid, bestehend aus:
(A) einem Peptid, das in der Länge aus 5 bis 10 Resten besteht, die innerhalb einer Aminosäuresequenz der SEQ ID NR.1 enthalten sind, umfassend ein Alanin (Ala), das an der fünften Position von dem N-Terminus in einer Aminosäuresequenz der SEQ ID NR. 1 gelegen ist, oder
(B) einem Peptid, das aus einer Aminosäuresequenz der SEQ ID NR. 2 besteht.

2. S100A8-hemmendes Peptid nach Anspruch 1, wobei das Peptid (A) aus einer Aminosäuresequenz besteht, die ausgewählt ist aus den SEQ ID NR. 1 und 3 bis 12.

3. S100A8-hemmendes Peptid nach Anspruch 1, wobei das Peptid (A) aus der Aminosäuresequenz der SEQ ID NR. 1 besteht.

4. Bivalentes S100A8-hemmendes Peptid, das zwei identische Peptide unter den Peptiden (A) und (B) enthält:
(A) einem Peptid, das in der Länge aus 5 bis 10 Resten besteht, die innerhalb einer Aminosäuresequenz der SEQ ID NR.1 enthalten sind, umfassend ein Alanin (Ala), das an der fünften Position von dem N-Terminus in einer Aminosäuresequenz der SEQ ID NR. 1 gelegen ist,
(B) einem Peptid, das aus einer Aminosäuresequenz der SEQ ID NR. 2 besteht.

5. Tetravalentes S100A8-hemmendes Peptid, das vier identische Peptide unter den Peptiden (A) und (B) enthält:
(A) einem Peptid, das in der Länge aus 5 bis 10 Resten besteht, die innerhalb einer Aminosäuresequenz der SEQ ID NR.1 enthalten sind, umfassend ein Alanin (Ala), das an der fünften Position von dem N-Terminus in einer Aminosäuresequenz der SEQ ID NR. 1 gelegen ist,
(B) einem Peptid, das aus einer Aminosäuresequenz der SEQ ID NR. 2 besteht.

6. Krankheitstherapeutisches Mittel, das das S100A8-hemmende Peptid nach einem der Ansprüche 1 bis 5 umfasst.

7. Krankheitstherapeutisches Mittel nach Anspruch 6, wobei die Krankheit mindestens eines ist von metastierendem Krebs, psychiatrischen Krankheiten, Zivilisationskrankheiten, rheumatischer Arthritis, COVID-19, Morbus Crohn, Colitis ulcerosa, zystischer Fibrose und allergischer Dermatitis.

## Revendications

1. Peptide inhibiteur de S100A8 consistant en :
(A) un peptide consistant en 5 à 10 résidus de long contenu dans une séquence d'acides aminés de SEQ ID NO : 1, comprenant une alanine (Ala) située à la cinquième position depuis la terminaison N dans une séquence d'acides aminés de SEQ ID NO : 1, ou
(B) un peptide consistant en une séquence d'acides aminés de SEQ ID NO : 2.

2. Peptide inhibiteur de S100A8 selon la revendication 1, dans lequel le peptide (A) consiste en une séquence d'acides aminés sélectionnée parmi les SEQ ID NO : 1 et 3 à 12.

3. Peptide inhibiteur de S100A8 selon la revendication 1, dans lequel le peptide (A) consiste en la séquence d'acides aminés de SEQ ID NO : 1.

4. Peptide inhibiteur de S100A8 bivalent contenant deux peptides identiques parmi les peptides (A) et (B) :
(A) un peptide consistant en 5 à 10 résidus de long contenu dans une séquence d'acides aminés de SEQ ID NO : 1, comprenant une alanine (Ala) située à la cinquième position depuis la terminaison N dans une séquence d'acides aminés de SEQ ID NO : 1,
(B) un peptide consistant en une séquence d'acides aminés de SEQ ID NO : 2.

5. Peptide inhibiteur de S100A8 tétravalent contenant quatre peptides identiques parmi les peptides (A) et (B) :
(A) un peptide consistant en 5 à 10 résidus de long contenu dans une séquence d'acides aminés de SEQ ID NO : 1, comprenant une alanine (Ala) située à la cinquième position depuis la terminaison N dans une séquence d'acides aminés de SEQ ID NO : 1,
(B) un peptide consistant en une séquence d'acides aminés de SEQ ID NO : 2.

6. Agent thérapeutique contre une maladie, comprenant le peptide inhibiteur de S100A8 selon l'une quelconque des revendications 1 à 5.

7. Agent thérapeutique contre une maladie selon la revendication 6, dans lequel la maladie est au moins l'un parmi un cancer métastatique, des maladies psychiatriques, des maladies de mode de vie, la polyarthrite rhumatoïde, le COVID-19, la maladie de Crohn, la colite ulcéreuse, la fibrose kystique et une dermatite allergique.
